Europäisches Patentamt

European Patent Office

Office européen des brevets

(19)

(11) Veröffentlichungsnummer: **0 224 608**
**B1**

(12) # EUROPÄISCHE PATENTSCHRIFT

(45) Veröffentlichungstag der Patentschrift:
20.06.90

(51) Int. Cl.⁵: **A61F 9/02**

(21) Anmeldenummer: **85115332.0**

(22) Anmeldetag: **03.12.85**

(54) **Schutzbrille.**

(43) Veröffentlichungstag der Anmeldung:
**10.06.87 Patentblatt 87/24**

(45) Bekanntmachung des Hinweises auf die Patenterteilung:
**20.06.90 Patentblatt 90/25**

(84) Benannte Vertragsstaaten:
**AT BE CH DE FR GB IT LI LU NL SE**

(56) Entgegenhaltungen:
**GB-A- 573 676**
**GB-A- 573 676**
**US-A- 1 670 638**
**US-A- 2 085 844**
**US-A- 3 141 172**
**US-A- 4 027 342**

(73) Patentinhaber: **UVEX WINTER OPTIK GMBH,**
**Salzstrasse 18-22, D-8510 Fürth 2(DE)**

(72) Erfinder: **Wiedner, Klaus, Dipl.-Kaufm.,**
**Coubertinstrasse 28, D-8510 Fürth/Bay(DE)**

(74) Vertreter: **Rau, Manfred, Dr. Dipl.-Ing. et al, Rau &**
**Schneck, Patentanwälte Königstrasse 2,**
**D-8500 Nürnberg 1(DE)**

ACTORUM AG

## Beschreibung

Die Erfindung richtet sich auf eine Schutzbrille gemäß dem Oberbegriff von Anspruch 1.

Derartige Schutzbrillen, welche in der Regel als Vollsicht-Brillen bezeichnet werden, finden Verwendung z.B. als Arbeitsschutzbrillen oder als Skibrillen. Bei solchen Brillen ist es bekannt, in den Rahmen selbst Belüftungsöffnungen einzubringen, um ein Beschlagen der Scheiben möglichst zu unterbinden. Die zur Festlegung derartiger Schutzbrillen dienenden Halteriemen werden an Befestigungseinrichtungen angebracht, welche entweder an den Rahmen angespritzt sind oder in relativ aufwendiger Weise dort montiert werden müssen.

Aus der US-A 1 670 638 und der GB-A 573 676 sind Schutzbrillen bekannt, bei welchen die Befestigungseinrichtungen für den Halteriemen gleichzeitig auch dazu dienen, eine Belüftung des Brilleninnenraums zu bewerkstelligen. Die US-A 3 141 172 beschreibt eine Schutzbrille, bei welcher gesondert von der Befestigungseinrichtung für den Halteriemen Belüftungseinrichtungen vorgesehen sind, welche zweiteilig ausgebildet sind und deren beide Teile durch eine Ausnehmung des Brillenraumes hindurch miteinander verschraubt werden.

Der Erfindung liegt die Aufgabe zugrunde, eine Schutzbrille der eingangs genannten Art so auszugestalten, daß sie bei spritztechnisch einfacher Herstellbarkeit und bei geringem Montageaufwand für die Befestigungseinrichtungen des Halteriemens gleichzeitig eine gute Belüftbarkeit des Brilleninnenraums gewährleistet.

Diese Aufgabe wird gelöst durch die Merkmale des kennzeichnenden Teils von Anspruch 1. Eine derart ausgestaltete Schutzbrille ermöglicht herstellungstechnisch und konstruktiv einfach eine zuverlässige Belüftung und gleichzeitig eine Befestigung des Halteriemens.

Vorteilhafte Ausgestaltungen ergeben sich aus den Unteransprüchen.

Weitere Merkmale, Vorteile und Einzelheiten der Erfindung ergeben sich aus der folgenden Beschreibung einer bevorzugten Ausführungsform anhand der Zeichnung. Dabei zeigen:

Fig. 1 eine teilweise perspektivische Ansicht einer erfindungsgemäßen Schutzbrille, welche im Bereich der Befestigungseinrichtung teilweise aufgebrochen ist,

Fig. 2 einen teilweisen Schnitt durch den Anbringungsbereich einer Befestigungeinrichtung und

Fig. 3 eine Explosionsdarstellung der Teile einer Befestigungseinrichtung.

Eine in der Zeichnung dargestellte Schutzbrille, z.B. eine Arbeitsschutzbrille, umfaßt eine Sichtscheibe 1 und einen Rahmen 2 aus elastisch deformierbarem Kunststoff. An dem Rahmen 2 ist mittels Befestigungseinrichtungen 3, wovon lediglich eine in der Zeichnung dargestellt ist, ein Halteriemen 4 festgelegt.

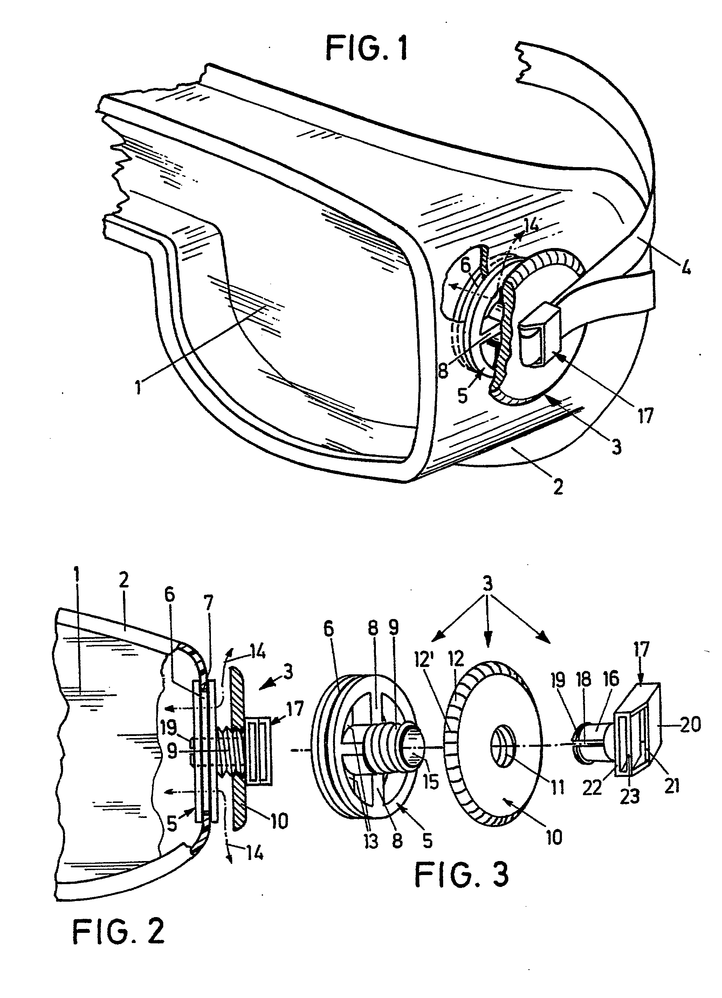

Jede Befestigungseinrichtung 3 umfaßt eine Grundplatte 5. Die kreisförmige Grundplatte 5 ist ebenso wie alle anderen Teile jeder Befestigungseinrichtung 3 aus Kunststoff gespritzt. Sie weist eine umlaufende Nut 6 auf. Mit Hilfe dieser Nut kann die Grundplatte 5 und damit die Befestigungseinrichtung 3, wie insbesondere in Fig. 2 dargestellt, in eine korrespondierende, kreisscheibenförmige Ausnehmung 7 des Rahmens 2 formschlüssig eingesetzt, d.h. eingeknöpft werden.

Die Grundplatte 5 weist weiterhin Stege 8 auf, im Ausführungsbeispiel 4 derartige Stege, welche einen zentralen, sich im eingesetzten Zustand nach außen erstreckenden Gewindeansatz 9 tragen. Auf diesem Gewindeansatz 9 ist eine Abdeckplatte 10 aufschraubbar, welche eine Gewindebohrung 11 aufweist. Die Abdeckplatte 10 weist eine kreisscheibenförmige Grundform mit einem etwas größeren Durchmesser als die Grundplatte 5 auf. Der Außenrand 12 der Abdeckplatte 10 ist mit einer Riffelung 12' versehen, welche das Verdrehen der Abdeckplatte 10 erleichtert.

In der Grundplatte 5 sind zwischen den Stegen 8 freie Durchbrechungen ausgebildet, welche als Belüftungsöffnungen 13 dienen. Durch die Abdeckplatte 10 können diese Belüftungsöffnungen 13 völlig abgedeckt werden, wenn die Abdeckplatte 10 vollständig auf den Gewindeansatz 9 aufgeschraubt ist, wobei andererseits durch ein Verschrauben in entgegengesetzter Richtung die Belüftungsöffnungen 13 veränderbar geöffnet werden können. Durch die Pfeile 14 in Fig. 2 wird veranschaulicht, daß die Zuluft nicht direkt durch die Belüftungsöffnungen 13 eindringen kann, so daß vermieden wird, daß der Träger der Brille ein Zuggefühl empfindet.

Der Gewindeansatz 9 bzw. die Grundplatte 5 weisen eine zentrale Bohrung 15 auf. In diese Bohrung 15 ist ein Zapfenansatz 16 einer Umlenkeinrichtung 17 für den Halteriemen 4 einsetzbar. Der Zapfenansatz 16 weist Längsschlitze 18 auf und ist an seinem vorderen freien Ende mit einem umlaufenden Rastwulst 19 versehen. Die eigentliche Umlenkeinrichtung wird gebildet durch einen kastenförmigen Grundkörper 20 mit zwei zueinander parallelen Außenwänden 21, 22 und einem Umlenksteg 23, um welchen der Halteriemen 4, wie in Fig. 1 dargestellt, geschlungen wird.

Die Umlenkeinrichtung wird an der Grundplatte 5 dadurch befestigt, daß der Zapfenansatz 16 in die Bohrung 15 eingeschoben wird, wobei der Zapfenansatz 16 aufgrund der Schlitze 18 zusammengedrückt wird. Nachdem der Rastwulst 19 nach dem Einschieben am anderen Ende der Bohrung 15 wieder austritt, wird er aufgrund der Eigenelastizität des für den Zapfenansatz 16 verwendeten Kunststoffmaterials nach außen gedrückt und übergreift, wie in Fig. 2 dargestellt, den Innenrand der Bohrung 15, so daß die Umlenkeinrichtung hierdurch axial festgelegt wird. Gleichzeitig bildet dann die Außenwand 22 der Umlenkeinrichtung 17, wie insbesondere aus Fig. 2 deutlich wird, einen Anschlag für die Abdeckplatte 10, wenn diese ganz nach außen geschraubt ist und die Belüftungsöffnungen 13 freigibt.

**Patentansprüche**

1. Schutzbrille mit einer Sicht-Scheibe (1) und einem diese aufnehmenden Rahmen (2) aus elastisch-deformierbarem Material, wobei an den Außenseiten des Rahmens (2) Befestigungseinrichtungen (3) für einen Halteriemen (4) angeordnet sind, und wobei die Befestigungseinrichtungen (3) mit dem Rahmen (2) formschlüssig verbindbar sind und Belüftungsöffnungen (13) für den Brillen-Innenraum aufweisen, dadurch gekennzeichnet, daß die Befestigungseinrichtungen (3) eine Grundplatte (5) aufweisen, wobei die Belüftungsöffnungen (13) als Durchbrechungen dieser Grundplatte (5) ausgebildet sind, und wobei die Grundplatte (5) einen zentralen Gewindeabschnitt (9) aufweist, auf welchen eine Abdeckplatte (10) für die Belüftungsöffnungen (13) derart aufschraubbar ist, daß sie die Belüftungsöffnungen (13) nach Wunsch absperrt oder freiläßt und dadurch eine Regulierung der Belüftung ermöglicht, und wobei der Gewindeabschnitt (9) und die Grundplatte (5) eine zentrale Bohrung (15) aufweisen, in welche ein Zapfenansatz (16) einer Umlenkeinrichtung (17) für den Halteriemen (4) einsetzbar ist.

2. Schutzbrille nach Anspruch 1, dadurch gekennzeichnet, daß die Befestigungseinrichtungen (3) eine umlaufende Nut (6) aufweisen und in eine korrespondierende Ausnehmung (7) des Rahmens (2) derart einknöpfbar sind, daß der Rand der Ausnehmung (7) in der Nut (6) zu liegen kommt.

3. Schutzbrille nach Anspruch 1, dadurch gekennzeichnet, daß die Abdeckplatte (10) kreisscheibenförmig ausgebildet ist und einen geriffelten Randbereich (12) aufweist.

4. Schutzbrille nach Anspruch 1, dadurch gekennzeichnet, daß der Zapfenansatz (16) in die zentrale Bohrung (15) einrastbar ist.

**Claims**

1. Protective goggles having a lens (1) and a frame (2) for receiving same, the frame made of elastically deformable material, having securing devices (3) for a fastening strap (4) disposed on the outsides of the frame (2), wherein the securing devices (3) can be joined in a form-fitting manner to the frame (2) and have vent openings (13) for the interior of the goggles, characterized in that the securing devices (3) have a base plate (5), wherein the vent openings (13) are perforations of this base plate (5), and wherein the base plate (5) has a central threaded portion (9), onto which a covering (10) for the vent openings (13) can be screwed in such a manner that it covers or opens the vent openings as desired thus allowing a control of the ventilation, and wherein the threaded portion (9) and the base plate (5) have a central bore (15) into which a protrusion (16) of a swivelling device (17) for the fastening strap (4) can be inserted.

2. Protective goggles as defined in patent claim 1, characterized in that the securing devices (3) have an encircling groove (6) and can be snapped into a corresponding recess (7) of the frame (2) in such a manner that the rim of the recess (7) comes to rest in the groove (6).

3. Protective goggles as defined in patent claim 1, characterized in that the covering plate (10) is in the form of a circular disk and has a ribbed rim area (12).

4. Protective goggles as defined in patent claim 1, characterized in that the protrusion (16) can be snapped into the central bore (15).

**Revendications**

1. Lunettes de protection comportant un verre de vision (1) et une monture (2) pour celui-ci en matériau élastiquement déformable, des dispositifs de fixation (3) pour une courroie de retenue (4) étant disposés sur le côté extérieur de la monture, les dispositifs de fixation (3) pouvant être assemblés en liaison par forme avec la monture (2) et présentant des ouvertures de ventilation (13) pour le volume intérieur des lunettes, caractérisées en ce que les dispositifs de fixation (3) comportent une plaque de base (5), les ouvertures de ventilation (13) étant constituées sous forme de lumières dans cette plaque de base (5), la plaque de base (5) présentant une partie filetée centrale (9) sur laquelle une plaque de recouvrement (10) des ouvertures de ventilation (13) peut être vissée de façon à fermer et à ouvrir à volonté ces ouvertures de ventilation (13) en permettant ainsi un réglage de la ventilation, la partie filetée (9) et la plaque de base (5) présentant un alésage central (15) dans lequel peut s'engager le téton (16) d'un organe de renvoi (17) de la courroie de retenue (4).

2. Lunette de protection selon la revendication 1, caractérisées en ce les dispositifs de fixation (3) présentent une gorge périphérique (6) et peuvent être enclenchés dans un évidemment correspondant (7) de la monture (2) de manière que le bord de l'évidement (7) vienne reposer dans la gorge (6).

3. Lunette de protection selon la revendication 1, caractérisées en ce que la plaque de recouvrement (10) est réalisée en forme de disque circulaire et présente une zone de bordure striée (12).

4. Lunettes de protection selon la revendication 1, caractérisées en ce que le téton (16) peut s'enclencher dans l'alésage central (15).

# FIG. 1

# FIG. 2

# FIG. 3